# EUROPÄISCHE PATENTSCHRIFT

(11) **EP 0 794 166 B1**
(45) Veröffentlichungstag und Bekanntmachung des Hinweises auf die Patenterteilung: **08.12.1999**
(21) Anmeldenummer: 97100500.4
(22) Anmeldetag: 15.01.1997
(51) Int. Cl.: C07C 29/141, C07C 31/133

(54) **Verfahren zur Herstellung von Hydroxymethylcyclopropan**
Method for the preparation of hydroxymethylcyclopropane
Procédé pour la préparation d'hydroxyméthylcyclopropane

(30) Priorität: 07.03.1996 DE 19608852
(43) Veröffentlichungstag der Anmeldung: 10.09.1997
(73) Patentinhaber: Degussa-Hüls Aktiengesellschaft, 60287 Frankfurt am Main (DE)
(72) Erfinder: Kleemiss, Wolfgang, Dr., 45721 Haltern (DE); Feld, Marcel, Dr., 51147 Köln (DE)

(56) Entgegenhaltungen:
- DE-A- 3 538 132
- US-A- 5 475 151

## Beschreibung

Die Erfindung betrifft ein Verfahren zur Herstellung von Hydroxymethylcyclopropan (auch Cyclopropylmethanol genannt) durch katalytische Hydrierung von Formylcyclopropan. Hydroxymethylcyclopropan ist ein wertvolles Zwischenprodukt, beispielsweise für die Herstellung von Bakteriziden, Fungiziden, Herbiziden und Insektiziden.

Es ist bekannt, daß sich Hydroxymethylcyclopropan aus Cyclopropancarbonsäure durch Reduktion mittels Lithiumalanat herstellen läßt (Beilstein, Handbuch der Organischen Chemie, E6 IV,4). Bei anderen bekannten Verfahren setzt man Cyclopropancarbonsäure mit metallorganischen Verbindungen in Gegenwart von elektrophilen Verbindungen um (US-A-4 085 273, US-A-4 054 480, US-A-3 998 889, US-A-3 959 324). Nach J. Am. Chem. Soc., 82, 2645-2646 (1960) erhält man Hydroxymethylcyclopropan durch anodische Oxidation von Cyclobutancarbonsäure. All diese Verfahren sind aufwendig, bedürfen komplizierter und teurer Chemikalien, die zudem teilweise nicht ungefährlich handhabbar sind, und liefern nur mäßige Ausbeuten.

Einen wesentlichen Fortschritt stellt demgegenüber die Herstellung durch katalytische Hydrierung von Cyclopropancarbonsäureestern (DE-A1-35 38 132) dar. Als Katalysator dient dabei Zinkchromit, während Kupferchromit unter ähnlichen Bedingungen unter Öffnung des Cyclopropanringes in einer Ausbeute von 59 % n-Butanol ergibt.

Ein Nachteil dieses Verfahrens ist der relativ teure Ausgangsstoff, der in einer dreistufigen Synthese aus Butyrolakton hergestellt wird (DE-A-29 41 211, EP-A-0 220 412).

Aus US 5 475 151 ist die Herstellung von Hydroxymethylcyclopropan durch Hydrierung von Formylcyclopropan an Kobalt- oder Nickelkatalysatoren bekannt.

Die vorliegende Erfindung löst die Aufgabe, Hydroxymethylcyclopropan aus einem leicht zugänglichen Ausgangsstoff in guten Ausbeuten und mit hoher Selektivität herzustellen. Die Aufgabe wird dadurch gelöst, daß Formylcyclopropan an Zinkchromit, Kupferchromit und/oder Kupfer/Zink-Katalysatoren bei 20 bis 250 °C hydriert wird.

Die für die Überführung der Carbonylfunktion in die Hydroxylfunktion in der Technik meistens verwendeten Edelmetallkatalysatoren, z.B. Palladium oder Platin, scheiden für die Hydrierung des Formylcyclopropans aus, da sie unter Öffnung des Cyclopropanrings zu offenkettigen Verbindungen führen (Houben-Weyl, Methoden der Organischen Chemie, IV/1c,405[1980]). So wird z.B. Cyclopropylmethylketon unter hydrierenden Bedingungen an Palladium glatt in 2-Pentanon überführt (J.Org.Chem. 36,383[1971]). Überraschenderweise erhalt bei dem Verfahren nach der Erfindung mit Kupferchromit und Kupfer/Zink als Katalysator Ausbeuten von über 90%, obwohl Cyclopropancarbonsäureester an Kupferchromit, wie zuvor erwähnt, weit überwiegend unter Ringöffnung zu n-Butanol hydriert werden. Überraschend ist weiterhin, daß die Hydrierung an Kupferchromit bereits bei Temperaturen unterhalb von 40°C bei vollständigem Umsatz mit sehr guten Ausbeuten und entsprechend hoher Selektivität in bezug auf Hydroxymethylcyclopropan gelingt. Ein Vorteil des Verfahrens nach der Erfindung gegenüber der Hydrierung von Cyclopropancarbonsäureestern besteht weiterhin darin, daß kein Alkohol entsteht, was die Aufarbeitung des Hydrierungsgemisches erleichtert.

Formylcyclopropan kann aus 1,4-Butandiol über 2,3-Dihydrofuran als Zwischenstufe (US-A-4 275 238) oder aus 1,3-Butadien über die Zwischenstufen 1,3-Butadienmonoepoxid, 2,5-Dihydrofuran und 2,3-Dihydrofuran hergestellt werden (US-A-5 138 077, US-A-5 315 019, DE-A1-28 03 987). Es handelt sich um katalytische Verfahren, die ohne Hilfsstoffe in stöchiometrischen Mengen auskommen und daher, obwohl mehrstufig, ein preiswertes Formylcyclopropan ergeben.

Die für die Erfindung verwendeten Katalysatoren sind an sich bekannt, mehrere sind im Handel erhältlich. Geeignete Zinkchromit-Katalysatcren enthalten im allgemeinen 40 bis 80 Gew.-% ZnO sowie 20 bis 40 Gew.-% Cr₂O₃ und haben einen Glühverlust von 10 bis 20 Gew.-%. Ein geeigneter Zinkchromitkatalysator ist z.B. der Katalysator S5-10 der BASF AG. Die Zinkchromit-Katalysatoren können auch andere Metalloxide enthalt, ohne daß die gewünschte Aktivität abnimmt, z.B. 1 bis 20 Gew.-%, bezogen auf die Summe von ZnO und Cr₂O₃, an Oxiden des Aluminiums, Eisens, Titan, Mangans und/oder der seltenen Erden sowie der Alkali- und Erdalkalimetalle.

Geeignete Kupferchromit-Katalysatoren enthalten im allgemeinen 30 bis 60 Gew.-% CuO sowie 30 bis 60% Cr₂O₃ und haben einen Glühverlust von wiederum 10 bis 20 Gew.-%. Ein geeigneter Kupferchromit-Katalysator ist z.B. der Mallinckrodt-Katalysator E406TU. Auch die Kupferchromit-Katalysatoren können weitere Metalloxide enthalten, ohne daß die gewünschte Aktivität abnimmt, z.B. die bei der Beschreibung des Zinkchromit-Katalysators genannten Metalloxide in den dort genannten Mengen.

Geeignete Kupfer/Zink-Katalysatoren können z.B. durch Mischfällung aus Kupfer- und Zinksalze enthaltenden Lösungen. Waschen der abgetrennten Fällung mit Wasser, Trocknen der abgetrennten Fällung und Umwandlung der so erhaltenen oxidischen Form des Katalysators in die hydrieraktive reduzierte Form durch Erhitzen in einer Wasserstoffatmosphäre hergestellt werden. Die Katalysatoren enthalten in ihrer oxidischen Form im allgemeinen 30 bis 50 Gew.-% CuO sowie 20 bis 40 Gew.-% ZnO und haben einen Glühverlust von 20 bis 40 Gewichtsprozent. Sie können wiederum die zuvor beispielhaft genannten anderen Metalloxide in den beispielhaft aufgeführten Mengen enthalten. Die oxidische Form des Katalysators kann durch Behandlung mit Wasserstoff, z.B. bei 200 bis 400°C unter Atmosphärendruck oder erhöhtem Druck, wie 50 bar, in die hydrieraktive reduzierte Form überführt werden.

Die Hydrierung nach der Erfindung wird zweckmäßig bei 20 bis 250°C durchgeführt. Unterhalb von 20°C ist die Hydriergeschwindigkeit auch der aktivsten Katalysatoren gering. Oberhalb von 250°C nehmen Zersetzungsreaktionen selbst bei entsprechend kurzen Kontaktzeiten rasch zu. Innerhalb des genannten Temperaturbereichs liegen die optimalen Temperaturen für Zinkchromit-Katalysatoren bei 180 bis 220 °C. diejenigen für Kupferchromit-Katalysatoren bei 30 bis 50 °C und diejenigen für Kupfer/Zink-Katalysatoren bei 80 bis 150 °C.

Der Wasserstoffdruck beträgt bei dem Verfahren nach der Erfindung, unabhängig von dem verwendeten Katalysator, im allgemeinen 10 bis 350 bar, vorteilhaft 100 bis 320 bar und insbesondere 150 bis 300 bar.

Das Formylcyclopropan kann bei dem Verfahren nach der Erfindung ohne Lösungs- oder Verdünnungsmittel eingesetzt werden. Gegebenenfalls können z.B. Alkohole, Ether, Alkane, Aromaten und Alkylaromaten als Lösungs- oder Verdünnungsmittel mitverwendet werden. Wegen der überraschend hohen thermischen Stabilität des Zielprodukts Hydroxymethylcyclopropan ist es möglich, dieses Zielprodukt selbst als Lösungs- oder Verdünnungsmittel zu verwenden.

Das Verfahren nach der Erfindung kann diskontinuierlich und besonders vorteilhaft kontinuierlich durchgeführt werden. Bei der diskontinuierlichen Arbeitsweise verwendet man üblicherweise einen Druckautoklaven mit Temperaturregelung und arbeitet in der Sumpfphase mit einem pulverförmigen Katalysator. Man bringt das Ausgangsgemisch auf die Reaktionstemperatur und preßt Wasserstoff nach, bis der Druck konstant bleibt. Eine Hydrierung dauert auf diese Weise etwa 3 bis 10 Stunden.

Bei einer kontinuierlichen Arbeitsweise bedient man sich vorteilhaft des Rieselverfahrens, bei dem man den Ausgangsstoff über den in einem beheizbaren Reaktionsrohr angeordneten, grobstückigen Katalysator rieseln läßt, während man Wasserstoff im Gleich- oder im Gegenstrom führt. Vorteilhaft wird überschüssiger Wasserstoff im Kreis geführt. Die LHSV (liquid hourly space velocity) beträgt, je nach Katalysator und Temperatur, im allgemeinen 0.1 bis 0.75 h⁻¹. In beiden Fällen wird das Reaktionsprodukt zweckmäßig durch diskontinuierliche oder kontinuierliche fraktionierte Destillation aufgearbeitet.

Die folgenden Beispiele sollen die Erfindung erläutern, nicht aber deren Anwendungsbereich begrenzen, wie er in den Patentansprüchen dargelegt ist.

### Beispiel 1

Ein senkrecht stehender 400ml Rohrreaktor wurde mit Kupferchromit-Katalysator (E406TU von Mallinckrodt) mit einer Stückgröße von 5 bis 10 mm gefüllt. Über den Katalysator ließ man stündlich 200 ml Formylcyclopropan rieseln, wobei die Temperatur im Reaktionsrohr auf 40°C gehalten wurde. Durch das Rohr wurde gleichzeitig im Gleichstrom Wasserstoff unter einem Druck von 285 bar geleitet. Die LHSV betrug 0.125 h⁻¹.

Das Hydrierungsprodukt wurde über eine 20 cm lange Füllkörperkolonne destilliert. Man erhielt, bezogen auf das Hydrierungsgemisch:

| | |
|---|---|
| Vorlauf | 5 Gew.-% |
| Hydroxymethylcyclopropan | 91 Gew.-% |
| Nachlauf | 4 Gew.-% |

Der Umsatz an Formylcyclopropan betrug >99%. die Ausbeute an Hydroxymethylcyclopropan entspricht 91 %d.Th.

### Beispiel 2

Formylcyclopropan wurde im wesentlichen nach der Arbeitsweise des Beispiels 1 an einem Zinkchromit-Katalysator (S5-10 von BASF AG) mit einer Stückgröße von 5 bis 10 mm hydriert. Die Temperatur betrug 200°C, der Wasserstoffdruck 285 bar.

Der Hydrieraustrag wurde wiederum über eine 20 cm lange Füllkörperkolonne destilliert. Man erhielt, bezogen auf das Hydrierungsgemisch:

| | |
|---|---|
| Vorlauf | 9 Gew.-% |
| Hydroxymethylcyclopropan | 61 Gew.-% |
| Nachlauf | 30 Gew.-% |

Der Umsatz des Formylcyclopropans betrug wiederum >99%, die Ausbeute an Hydroxymethylcyclopropan ca. 61%.

### Beispiel 3

Formylcyclopropan wurde im wesentlichen nach der Arbeitsweise des Beispiels 1 an einem Kupfer/Zink-Katalysator mit einer Stückgröße von 5 bis 10 mm hydriert. Der Katalysator hatte in der oxidischen Form die folgende Zusammensetzung: 42,2 Gew.-% CuO, 26,9 Gew.-% ZnO, 30,9 Gew.-% Glühverlust. Er wurde in dieser Form in den Reaktor eingefüllt und durch Überleiten von Wasserstoff unter einem Druck von 1 bar bei einer Temperatur von 250°C innerhalb von 6 Stunden in die reduzierte, aktive Form überführt. Das Formylcyclopropan wurde bei einer Temperatur von 120°C und einem Wasserstoffdruck von 285 bar hydriert.

Der Hydrieraustrag wurde wiederum über eine 20 cm lange Füllkörperkolonne destilliert. Man erhielt, bezogen auf das Hydrierungsgemisch:

| | |
|---|---|
| Vorlauf | 5 Gew.-% |
| Hydroxymethylcyclopropan | 90 Gew.-% |
| Nachlauf | 5 Gew.-% |

Der Umsatz des Formylcyclopropans betrug wiederum >99%, die Ausbeute an Hydroxymethylcyclopropan ca. 90%.

## Patentansprüche

1. Verfahren zur Herstellung von Hydroxymethylcyclopropan,
dadurch gekennzeichnet,
daß Formylcyclopropan an Kupferchromit, Zinkchromit und/oder Kupfer/Zink-Katalysatoren bei 20 bis 250 °C hydriert wird.

2. Verfahren nach Anspruch 1,
dadurch gekennzeichnet,
daß der Katalysator Zinkchromit ist und die Temperatur 180 bis 220 °C beträgt.

3. Verfahren nach Anspruch 1,
dadurch gekennzeichnet,
daß der Katalysator Kupferchromit ist und die Temperatur 30 bis 50 °C beträgt.

4. Verfahren nach Anspruch 1,
dadurch gekennzeichnet,
daß der Katalysator Kupferoxid/Zinkoxid ist und die Temperatur 80 bis 150 °C beträgt.

5. Verfahren nach einem der Ansprüche 1 bis 4,
dadurch gekennzeichnet,
daß der Wasserstoffdruck 10 bis 350 bar beträgt.

6. Verfahren nach einem der Ansprüche 1 bis 5,
dadurch gekennzeichnet,
daß das Verfahren in der Sumpf- oder in der Rieselphase durchgeführt wird.

## Claims

1. A process for preparing hydroxymethylcyclopropane, characterized in that formylcyclopropane is hydrogenated at from 20 to 250°C over copper chromite, zinc chromite and/or copper/zinc catalysts.

2. A process according to claim 1, characterized in that the catalyst is zinc chromite and the temperature is from 180 to 220°C,

3. A process according to claim 1, characterized in that the catalyst is copper chromite and the temperature is from 30 to 50°C,

4. A process according to claim 1, characterized in that the catalyst is copper oxide/zinc oxide and the temperature is from 80 to 150°C,

5. A process according to any one of claims 1 to 4, characterized in that the hydrogen pressure is from 10 to 350 bar.

6. A process according to any one of claims 1 to 5, characterized in that the process is carried out in the bottom phase or in the trickle phase.

## Revendications

1. Procédé de fabrication d'hydroxyméthylcyclopropane, caractérisé en ce que l'on effectue une hydrogénation de formylcyclopropane sur des catalyseurs de chromite de cuivre chromite de zinc et/ou cuivre/zinc, à des températures de 20 à 250°C.

2. Procédé selon la revendication 1,
caractérisé en ce que
le catalyseur est le chromite de zinc et la température est de 180 à 220°C.

3. Procédé selon la revendication 1,
caractérisé en ce que
la catalyseur est du chromite de cuivre et la température est de 30 à 50°C.

4. Procédé selon la revendication 1,
caractérisé en ce que
la catalyseur est de l'oxyde de cuivre/oxyde de zinc, et la température est de 80 à 150°C.

5. Procédé selon l'une des revendications 1 à 4,
caractérisé en ce que
la pression d'hydrogène est de 10 à 350 bars.

6. Procédé selon l'une des revendications 1 à 5,
caractérisé en ce que
le procédé est effectué dans la phase de distillation poussée (bas de colonne) ou bien dans la phase de ruissellement.
